# FASCICULE DE BREVET EUROPEEN

(11) **EP 0 420 743 B1**
(45) Date de publication et mention de la délivrance du brevet: **09.02.2000**
(21) Numéro de dépôt: 90402639.0
(22) Date de dépôt: 25.09.1990
(51) Int. Cl.: A61K 39/102

(54) **Vaccin protecteur contre l'hémophilose porcine**
Impfstoff zum Schutz gegen Schweinhämophilus
Vaccine protecting against porcine hemophilus

(30) Priorité: 26.09.1989 FR 8912567
(43) Date de publication de la demande: 03.04.1991
(73) Titulaire: MERIAL, 69002 Lyon (FR)
(72) Inventeur: Desmettre, Philippe, F-69130 Ecully (FR); Milward, Francis, F-69160 Tassin (FR); Prevost, Philippe, F-69004 Lyon (FR); Rolland,Eric, F-69007 Lyon (FR)
(74) Mandataire: Bernasconi, Jean

(56) Documents cités:
- WO-A-80/02113
- GB-A- 2 197 193
- COMMONWEALTH AGRICULTURAL BUREAUX, Castelar (AR); J.M. MIQUET et al., no. 0141079#
- COMMONWEALTH AGRICULTURAL BUREAUX, Castelar (AR); S. ROSENDAL et al., no. 0520089#
- R. HESSE, "Haemophilus Pleuropneumonia Vaccination/Challenge Studies", 1984, IPVS de Ghent, BE; p. 111#
- VACCINE, vol. 5, no. 4, décembre 1987, Londres (GB); INSTITUT PASTEUR BUCHAREST, p. 319#
- BIOLOGICAL ABSTRACT; L. MOLNAR et al., no. 84004412#
- CHEMICAL ABSTRACTS, vol. 105, no. 25, 22 décembre 1986, Columbus, OH (US); W.B. FENWICK et al., p. 581, no. 224115r#
- CHEMICAL ABSTRACTS, vol. 95, no. 1, 06 juillet 1981, Columbus, OH (US); p. 365, no. 12749a#
- CHEMICAL ABSTRACTS, vol. 95, no. 4, 27 juillet 1981, Columbus, OH (US); p. 339, no. 30390f#

## Description

L'invention concerne un vaccin protecteur contre l'hémophilose porcine et un procédé d'obtention du principe actif entrant dans la constitution dudit vaccin.

La pleuropneumonie du porc ou hémophilose porcine est une maladie largement répandue géographiquement et qui a de graves conséquences économiques. Cette maladie se traduit notamment par une pleurésie fibrineuse et par une pneumonie hémorragique.

Le germe responsable de cette maladie est Hemophilus (Actinobacillus)pleuropneumoniae que l'on appellera par la suite H. pleuropneumoniae. Douze sérotypes capsulaires sont décrits dans le monde.

Les facteurs de virulence d'H. pleuropneumoniae sont au nombre de trois : l'endotoxine (lipopolysaccharide LPS), la capsule (polysaccharide capsulaire) et une ou plusieurs toxine(s) responsable(s) de l'activité cytotoxique et de l'activité hémolytique d'H. pleuropneumoniae. Lesdites toxines sont encore mal définies et on ne sait pas avec certitude si ces deux activités sont le fait d'une seule ou de plusieurs toxine(s).

Devant les difficultés de mise en oeuvre des programmes d'assainissement, la vaccination a été souvent envisagée. A la suite de l'échec des vaccins préparés à partir de corps bactériens inactivés (protection faible et spécifique des sérotypes inclus dans le vaccin, mauvaise innocuité), des vaccins de type "sous-unité" ont été étudiés.

Cependant, les tentatives de préparation de vaccins sous-unité à partir du lipopolysaccharide, du polysaccharide capsulaire et de protéines de la membrane externe n'ont pas abouti : l'effet protecteur est insuffisant et spécifique.

On s'est intéressé à l'hémolysine (toxine hémolytlque) à la suite des travaux de Bendixen et al. (Toxicity of Haemophilus pleuropneumoniae for porcine lung macrophages, peripheral blood monocytes and testicular cells - Infect. Immum., 1981, 33, 673-676) et de Rosendal et al.(H. pleuropneumoniae lung lesions induced by sonicated bacteria and sterile culture supernatant - Proceedings IPVS Copenhagen, 1980, p. 221) qui ont montré la présence d'anticorps antihémolysine dans des sérums de porcs convalescents et l'effet neutralisant d'un sérum de lapin hyper-immun vis-à-vis des activités hémolytiques et cytotoxiques.

Van Leengoed et al. (Vaccination of pigs with toxin containing supernatant of H. pleuropneumoniae - 1988, Thesis "Pathogenic studies on H. pleuropneumoniae", State University of Utrecht) ont montré qu'un vaccin à base de toxlne du sérotype 9 préparé en adjuvant huileux conférait une protection lors d'une épreuve virulente homologue chez le porc. Les animaux vaccinés présentent des anticorps neutralisant les activités hémolytiques et cytotoxiques.

Par contre, R. Hesse et al. (H. pleuropneumoniae vaccination - Challenge studies, 1984, IPVS de Ghent, Belgique, p. 111) ont montré qu'un vaccin à base de cytotoxine du sérotype 1 inactivée par la chaleur et non adjuvée, ne conférait pas de protection lors d'une épreuve virulente homologue et qu'un vaccin composé de toxine du sérotype 1 adjuvée par une émulsion ne protégeait pas contre une épreuve par le sérotype 5. Ils ont démontré une protection pour un vaccin comprenant à la fois la toxine et des corps bactériens inactivés.

Enfin. J. Frey et al. (Biochemical and genetic characterization of Actinobacillus pleuropneumoniae hemolysin, 1988, IPVS de Rio de Janeiro, p. 79) ont montré l'importance de la présence de Ca⁺⁺ dans le milieu de culture pour l'expression de l'hémolysine chez certains sérotypes, notamment le sérotype 1. Le milieu de culture qu'ils ont utilisé est le Columbia Broth additionné d'IsoVitaleX et de NAD.

L'invention a pour objectif de fournir un vaccin contre l'hémophilose porcine qui confère une protection efficace contre le sérotype 1 et au moins partielle contre les autres sérotypes d'H. pleuropneumoniae et qui présente une grande innocuité.

Un autre objectif de l'invention est de fournir un vaccin peu onéreux et facile à fabriquer.

L'invention a pour objet un vaccin protecteur contre l'hémophilose porcine, comprenant l'anatoxine purifiée obtenue par inactivation, par le formol, de la toxine du sérotype 1 d'**Haemophilus** (**Actinobacillus**) **pleuropneumoniae**, qui est le support d'une activité cytotoxique et hémolytique d'**H. pleuropneumoniae** et qui apparaît sous la forme d'une bande à 105 KDa en électrophorèse PAGE-SDS, et un véhicule ou excipient du type de ceux utilisés dans la constitution de vaccins, ce vaccin étant apte, lorsqu'il est administré au porc, à induire une protection efficace contre **H. pleuropneumoniae**.

De préférence, l'anatoxine est adjuvée, notamment par de l'hydroxyde d'aluminium.

De préférence, l'anatoxine est à une concentration finale de 50 µg/ml.

De préférence, le vaccin est administré à raison de 100 µg d'anatoxine par dose.

De préférence, l'anatoxine entrant dans la constitution du vaccin est obtenue par purification du surnageant d'une culture d'une souche d'H. pleuropneumoniae de sérotype 1 fortement productrice de toxine, dans un milieu additionné de Ca⁺⁺ et de NAD.

Dans une forme de réalisation perfectionnée le vaccin selon l'invention peut comprendre, en plus de l'anatoxine du sérotype 1, ainsi définie, de l'anatoxine purifiée d'un autre sérotype, notamment 2, 3, 4, 6, 8 et 12 à partir de la toxine convenablement inactivée par le formol.

L'anatoxine de cet autre sérotype peut être obtenue par concentration et purification poussées d'un surnageant de culture du sérotype mais on peut aussi, surtout s'il s'agit du sérotype 2,l'obtenir par expression d'un vecteur recombinant génétique, et on entend également par anatoxine d'autre sérotype, par exemple 2, l'anatoxine recombinante ainsi que ses fractions, variantes, et peptides présentant des épitopes pertinents, ou encore par synthèse peptidique.

La concentration de l'anatoxine de l'autre sérotype, dans le vaccin est de préférence de l'ordre de 50 µg/ml et la dose administrée comprend également, de préférence, une quantité similaire à celle de l'anatoxine de sérotype 1.

Par anatoxine, d'un sérotype donné, dans le sens de l'invention, on entend aussi bien l'un quelconque, ou la totalité des polypeptides du surnageant de culture auxquels sont attribués des activités cytotoxiques et/ou hémolytiques. Toutefois pour le sérotype 1, l'anatoxine comprend ou est constituée par l'hémolysine et, pour le sérotype 2, s'il est présent, ou les autres sérotypes, on préfère qu'il contienne, ou soit constitué par, un polypeptide d'environ 120 KDa auquel on attribue une activité cytotoxique.

Le vaccin selon l'invention sera de préférence administré par voie intra-musculaire, sous-cutanée ou intradermique en une ou deux injections.

L'invention a également pour objet un procédé de préparation de vaccins protecteurs du type décrit ci-dessus, dans lequel l'on sélectionne une souche d'H. pleuropneumoniae de sérotype 1 fortement productrice de toxine, on cultive la souche sélectionnée dans des conditions optimales d'expression de toxine, on récupère le surnageant de culture et on extrait et purifie la toxine, puis on inactive la toxine par le formol.

Selon l'invention, on peut cultiver la souche sélectionnée dans notamment l'un des trois milieux de culture suivants, contenant du Ca⁺⁺ et du NAD :
- milieu Bouillon Coeur-Cervelle,
- milieu Bacto Columbia Broth,
- milieu de Wilkins-Chalgren.

L'invention va maintenant être décrite plus en détail à l'aide d'un procédé d'obtention de la toxine du sérotype 1 et à l'aide d'essais de vaccination chez la souris et chez le porc.

### I. Procédé d'obtention de toxine

On a choisi la souche de sérotype 1 décrite par Shope R.E. (1964), J. Exp. Med. 119, 357-368, en raison de sa production importante de toxine.

Le milieu de culture utilisé doit contenir du Ca⁺⁺ et du NAD. Les milieux Bouillon Coeur-Cervelle (BioMérieux), Bacto Columbia Broth (Difco) et Wilkins-Chalgren Oxoid conviennent notamment.

Dans ce procédé, on utilise le milieu Bacto Columbia Broth, additionné de 10 mM de CaCl₂ et de 15 mM de NAD.

On met une ampoule lyophilisée de la souche en culture dans 5 ml de milieu, pendant 18 heures à 37°C et sous agitation. On ensemence ensuite 100 ml de milieu et on cultive 6 heures à 37°C, sous agitation.

A ce stade, la culture peut être inactivée par addition de formaldéhyde à 1,6 mg/ml.

On centrifuge ensuite la culture à 7000 g pendant 20 minutes. On concentre le surnageant par précipitatlon au sulfate d'ammonium à 40% de saturation, pendant 30 minutes à 4°C et sous agitation. Le précipité obtenu après centrifugation à 10 000 g pendant 10 mn est repris en tampon TNC (Tris HCL 10 mM, NaCl 9%, CaCl₂ 10 mM).

La toxine peut aussi être concentrée par ultrafiltration (seuil de coupure : 10 000 Da). Le concentré peut être inactivé par la chaleur, 1 heure à 56°C.

La toxine est ensuite purifiée par deux chromatographies successives de filtration sur gel S200 HR (Pharmacia-LKB). Le domaine de fractionnement de ce gel se situe entre 50 000 et 250 000 pour des protéines globulaires. Les caractéristiques de la colonne sont les suivantes :

| | |
|---|---|
| Volume de gel | 190 ml |
| Hauteur | 95 cm |
| Section | 2 cm |
| Volume de dépôt | 10 ml |
| Débit | 20 ml/h |
| Tampon d'élution | Tris HCL 10 mM |
| | NaCl 9% |
| | CaCl₂ 10 mM |

Le profil en électrophorèse PAGE-SDS (selon Laemli, 1970, Cleavage of structural protein during the assembly of the head of bacteriophage T4, Nature 227, 680-685) montre une seule bande, qui correspond à un poids moléculaire de 105 KDa (figure 1). Il semble donc qu'il n'existe, pour le sérotype 1, qu'une seule toxine responsable des deux activités, cytotoxique et hémolytique. En tout cas, conformément à l'invention le vaccin inclut l'anatoxine de poids moléculaire 105 KDa, même si d'autres composants antigéniques peuvent, ou non, être présents.

### II. Préparation d'un vaccin

Le vaccin est préparé à partir de l'anatoxine purifiée obtenue comme ci-dessus. La formulation en anatoxine se fait sur la base du titre hémolytique avant inactivation (titre de 3 environ) et pourra aussi se faire selon d'autres tests (ELISA par exemple). L'anatoxine est adjuvée en gel d'alumine (0,7 mg/ml) et formolée à 1,6 mg/ml.

### III. Essai de vaccination de souris

Le vaccin cité ci-dessus a été testé lors d'une vaccination épreuve sur souris :
les souris reçoivent à JO 0,5 ml de vaccin par voie sous-cutanée. L'épreuve est réalisée à J21 par injection de 10 doses létales 50% par souris par voie intrapéritonéale sous 0,5 ml. Les résultats figurent dans le tableau 1 suivant :
les résultats sont exprimés en nombre de morts pour 10 souris éprouvées :

**TABLEAU 1**

| Sérotype d'épreuve | Témoin | Vaccin "anatoxine" |
|---|---|---|
| 1 | 9 | 0 |
| 2 | 4 | 0 |
| 3 | 9 | 3 |
| 4 | 8 | 3 |
| 5 | 5 | 0 |
| 6 | 5 | 0 |
| 7 | 9 | 2 |
| 8 | 7 | 1 |
| 9 | 5 | 0 |
| 10 | 10 | 2 |
| 11 | 10 | 3 |
| 12 | 10 | 3 |

Ceci démontre que le vaccin confère une protection efficace et hétérologue vis-à-vis de tous les sérotypes décrits dans le modèle souris, extrapolable à l'espèce porcine.

### IV. Essai de vaccination chez le porc

Deux groupes de porcelets EOPS (exempts d'organismes pathogènes spécifiques) sont élevés dans les mêmes conditions. L'un des groupes est vacciné à l'âge de 8 semaines et de 12 semaines. L'autre groupe sert de témoin.

Les deux groupes sont éprouvés à l'âge de 14 semaines avec le sérotype 9 (épreuve hétérologue) par voie intranasale (10⁴ germes).

Les résultats sont regroupés dans le tableau 2 suivant.

**TABLEAU 2**

| | Symptômes cliniques | Mortalité | Lésions pulmonaires |
|---|---|---|---|
| Groupe de porcs vaccinés (pour 8 porcs) | 0 | 0 | 1 (petits abcès) |
| Groupe témoin (pour 8 porcs) | 8 | 8 (en 48 h) | 8 |

Ces résultats démontrent bien l'efficacité du vaccin selon l'invention.

Pour préparer un vaccin contenant également l'anatoxine de sérotype 2, on peut procéder à une culture de la souche de ce sérotype dans des conditions identiques ou similaires à celles décrites.

Le surnageant de culture est centrifugé puis concentré par précipitation au sulfate d'ammonium, le précipité étant repris puis purifié par chromatographie par échange d'ions suivie d'une gel-filtration, ou par chromatographie sur gel d'hydroxy-apatite. L'inactivation de la toxine purifiée est faite de préférence au formol.

Pour les autres sérotypes tels que 3, 4, 6, 8 , 12, on peut procéder comme pour l'anatoxine de sérotype 1.

## Revendications

1. Vaccin protecteur contre l'hémophilose porcine, comprenant l'anatoxine purifiée obtenue par inactivation, par le formol, de la toxine du sérotype 1 d'**Haemophilus** (**Actinobacillus**) **pleuropneumoniae**, qui est le support d'une activité cytotoxique et hémolytique d'**H. pleuropneumoniae** et qui apparaît sous la forme d'une bande à 105 kDa en électrophorèse PAGE-SDS, et un véhicule ou excipient du type de ceux utilisés dans la constitution de vaccins, ce vaccin étant apte, lorsqu'il est administré au porc, à induire une protection efficace contre **H. pleuropneumoniae**.

2. Vaccin selon la revendication 1, caractérisé en ce que l'anatoxine est adjuvée par de l'hydroxyde d'aluminium.

3. Vaccin selon l'une des revendications 1 à 2, caractérisé en ce que la toxine est obtenue par purification du surnageant d'une culture d'une souche d'H. pleuropneumoniae de sérotype 1 fortement productrice de toxine, dans un milieu additionné de Ca⁺⁺ et de NAD.

4. Vaccin selon l'une quelconque des revendications 1 à 3, caractérisé en ce qu'il contient également une anatoxine purifiée inactivée par le formol d'un autre sérotype 2, 3, 4, 6, 8 et 12.

5. Vaccin selon la revendication 4, caractérisé en ce qu'il comprend l'anatoxine purifiée obtenue par inactivation de la toxine de 120 kDa du sérotype 2 d'**H. pleuropneumoniae**.

6. Vaccin selon l'une quelconque des revendications 1 à 3, caractérisé en ce qu'il contient également une anatoxine purifiée du sérotype 6.

7. Procédé de préparation d'un vaccin protecteur selon l'une des revendications 1 à 6, caractérisé en ce que l'on sélectionne une souche d'H. pleuropneumoniae de sérotype 1 fortement productrice de toxine, on cultive la souche sélectionnée dans des conditions optimales d'expression de toxine, on récupère le surnageant de culture et on extrait et purifie la toxine, puis, on inactive la toxine par le formol.

8. Procédé selon la revendication 7, caractérisé en ce qu'on cultive la souche sélectionnée avec un milieu Bacto Columbia Broth contenant du Ca⁺⁺ et du NAD.

## Claims

1. Protective vaccine against porcine haemophilosis, comprising the purified anatoxin obtained by inactivation, with formaldehyde, of the toxin of the serotype 1 of **Haemophilus (Actinobacillus) pleuropneumoniae**, which is the support for a cytotoxic and haemolytic activity of **H. pleuropneumoniae** and which appears in the form of a 105 kDa band in PAGE-SDS electrophoresis, and a vehicle or excipient of the type used in the make up of vaccines, this vaccine being capable, when administered to a pig, of inducing effective protection against **H. pleuropneumoniae**.

2. Vaccine according to claim 1, characterised in that aluminium hydroxide is used as adjuvant for the anatoxin.

3. Vaccine according to one of claims 1 to 2, characterised in that the toxin is obtained by purifying the supernatant of a culture of a strain of *H. pleuropneumoniae* of serotype 1 which is a strong toxin producer, in a medium to which Ca⁺⁺ and NAD have been added.

4. Vaccine according to any of claims 1 to 3, characterised in that it also contains a purified anatoxin, inactivated by formaldehyde, of another serotype 2, 3, 4, 6, 8 and 12.

5. Vaccine according to claim 4, characterised in that it comprises the purified anatoxin obtained by inactivation of the 120 kDa toxin of serotype 2 of *H. pleuropneumoniae.*

6. Vaccine according to any one of claims 1 to 3, characterised in that it also contains a purified anatoxin of serotype 6.

7. Process for preparing a protective vaccine according to one of claims 1 to 6, characterised in that a strain of *H. pleuropneumoniae* of serotype 1 which is a strong producer of toxin is selected, the strain selected is cultivated under optimum conditions for toxin expression, the supernatant is recovered from the culture and the toxin is extracted and purified, then the toxin is inactivated with formaldehyde.

8. Process according to claim 7, characterised in that the strain selected is cultivated with a Bacto Columbia Broth medium containing Ca⁺⁺ and NAD.

## Patentansprüche

1. Impfstoff zum Schutz gegen Schweinepleuropneumonie, umfassend das gereinigte Anatoxin, erhalten durch Formol-Inaktivierung des Toxins des Serotyps 1 von *Haemophilus (Actinobacillus) pleuropneumoniae*, das der Träger einer cytotoxischen und hämolytischen Aktivität von *H. pleuropneumoniae* ist und bei der SDS-PAGE-Elektrophorese als Bande bei 105 kDa auftritt, und ein Vehikel oder einen Exzipienten des Typs, der zur Herstellung von Impfstoffen verwendet wird, wobei der Impfstoff, wenn er an Schweine verabreicht wird, zur Induktion eines gegen *H. pleuropneumoniae* wirksamen Schutzes geeignet ist.

2. Impfstoff nach Anspruch 1, dadurch gekennzeichnet, daß das Anatoxin von Aluminiumhydroxid unterstützt wird.

3. Impfstoff nach einem der Ansprüche 1 bis 2, dadurch gekennzeichnet, daß das Toxin durch Reinigung des Überstands einer Kultur eines *H. pleuropneumoniae*-Stammes vom Serotyp 1, der das Toxin stark produziert, in einem mit Ca⁺⁺ und NAD angereicherten Medium erhalten wird.

4. Impfstoff nach einem der Ansprüche 1 bis 3, dadurch gekennzeichnet, daß er ebenfalls ein gereinigtes, Formol-inaktiviertes Anatoxin eines anderen Serotyps 2, 3, 4, 6, 8 und 12 enthält.

5. Impfstoff nach Anspruch 4, dadurch gekennzeichnet, daß er das gereinigte Anatoxin umfaßt, das durch Inaktivierung des 120 kDa großen Toxins des Serotyps 2 von *H. pleuropneumoniae* erhalten wird.

6. Impfstoff nach einem der Ansprüche 1 bis 3, dadurch gekennzeichnet, daß er ebenfalls ein gereinigtes Anatoxin des Serotyps 6 enthält.

7. Verfahren zur Herstellung eines Schutz-Impfstoffs nach einem der Ansprüche 1 bis 6, dadurch gekennzeichnet, daß ein *H. pleuropneumoniae*-Stamm des Serotyps 1 ausgewählt wird, der das Toxin stark produziert, der ausgewählte Stamm unter optimalen Bedingungen für die Expression des Toxins gezüchtet wird, der Kulturüberstand gewonnen wird und das Toxin extrahiert und gereinigt sowie Formol-inaktiviert wird.

8. Verfahren nach Anspruch 7, dadurch gekennzeichnet, daß der ausgewählte Stamm mit einem Bacto-Columbia-Brühe-Medium gezüchtet wird, das Ca⁺⁺ und NAD enthält.
